# EUROPEAN PATENT APPLICATION

(11) **EP 4 817 508 A1**
(43) Date of publication of application: **07.10.2026**
(21) Application number: 24897194.7
(22) Date of filing: 31.10.2024
(51) Int. Cl.: A61B 1/24, A61B 1/00, A61B 1/045, A61C 19/04

(54) **TOOTH ROW IMAGE GENERATION DEVICE, TOOTH ROW IMAGE GENERATION METHOD, AND PROGRAM**

(30) Priority: 29.11.2023 JP 2023201600
(71) Applicant: Panasonic Intellectual Property Management Co., Ltd., Kadoma-shi, Osaka 571-0057 (JP)
(72) Inventor: OHTSUKA, Yoshio, Kadoma-shi, Osaka 571-0057 (JP); HAMASAKI, Takeshi, Kadoma-shi, Osaka 571-0057 (JP)
(74) Representative: Novagraaf International SA
(86) International application number: PCT/JP2024/038825
(87) International publication number: WO 2025/115505

(57) **Abstract**

A dentition image generation device includes: an obtainer (51) that obtains a captured image obtained by capturing an image of a surface of dentition and dental plaque in an oral cavity irradiated with light including a blue-light wavelength range; a combiner (53) that generates a dentition image obtained by combining a plurality of dentition block images that are partial dentition images based on the captured image; and a processing unit (52) that performs white balance processing on the dentition image.

## Description

### [Technical Field]

The present disclosure relates to a dentition image generation device, a dentition image generation method, and a program.

### [Background Art]

Patent Literature (PTL) 1 discloses a device that includes an irradiation means including: an excitation light emitter that emits excitation light for causing dental plaque, a lesion area, and the like, to emit fluorescence; and an illumination light emitter that emits illumination light for illuminating the periphery of the dental plaque, the lesion area, and the like, and the irradiation means is capable of simultaneously emitting the illumination light and the excitation light. Furthermore, Patent Literature 1 discloses that, in order to enhance visibility of dental plaque, a lesion area, and the like, the amount of emitted light from the excitation light emitter is greater than the amount of emitted light from the illumination light emitter.

### [Citation List]

### [Patent Literature]

[PTL 1] PCT International Publication No. WO 2005/104926

### [Summary of Invention]

### [Technical Problem]

Incidentally, in a dentition image generation device that generates a dentition image, it is desired that a dentition image with less unnatural appearance be generated.

In view of this, the present disclosure provides a dentition image generation device, a dentition image generation method, and a program that are capable of generating a dentition image in which an unnatural appearance is reduced.

### [Solution to Problem]

A dentition image generation device according to one aspect of the present disclosure includes: an obtainer that obtains a captured image obtained by capturing an image of a surface of dentition and dental plaque in an oral cavity irradiated with light including a blue-light wavelength range; a combiner that generates a dentition image obtained by combining a plurality of dentition block images that are partial dentition images based on the captured image; and a processing unit that performs white balance processing on the dentition image.

A dentition image generation method according to one aspect of the present disclosure includes: obtaining a captured image obtained by capturing an image of a surface of dentition and dental plaque in an oral cavity irradiated with light including a blue-light wavelength range; generating a dentition image obtained by combining a plurality of dentition block images that are partial dentition images based on the captured image; and performing white balance processing on the dentition image.

A program according to an aspect of the present disclosure is a program for causing a computer to execute the above dentition image generation method.

### [Advantageous Effects of Invention]

One aspect of the present disclosure can realize a dentition image generation device, and the like, capable of generating a dentition image in which an unnatural appearance is reduced.

### [Brief Description of Drawings]

[FIG. 1]

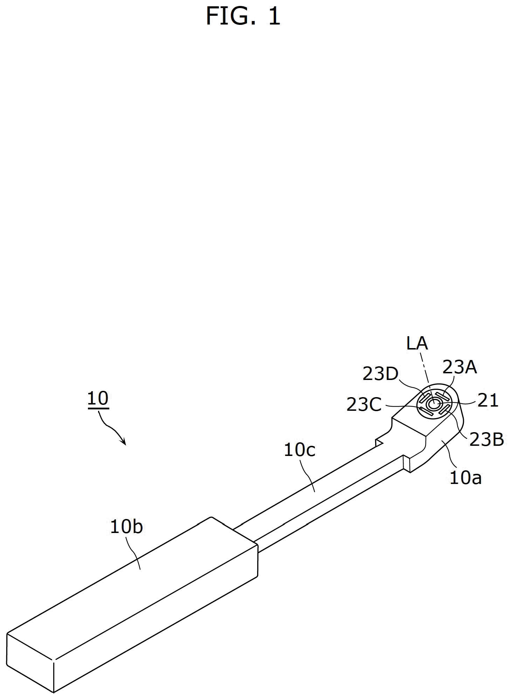

   FIG. 1 is a perspective view of an intraoral camera in a dentition image generation system according to an embodiment.
[FIG. 2]

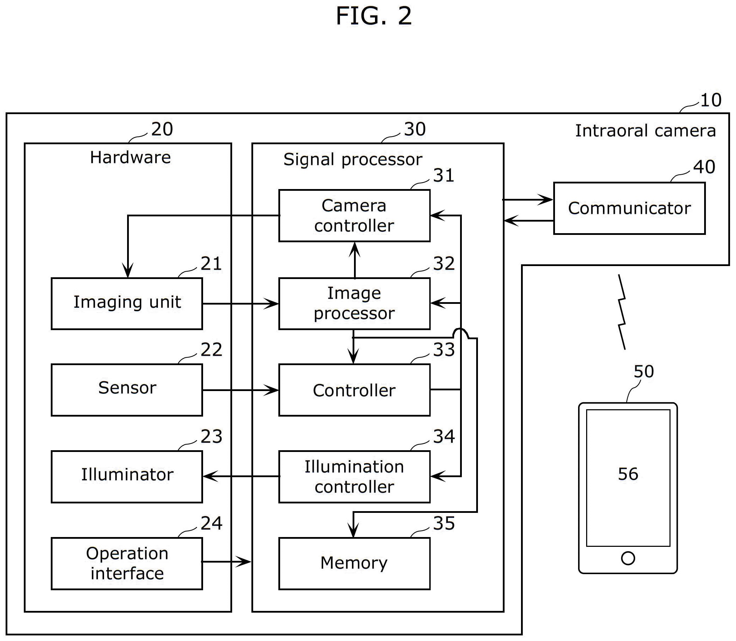

   FIG. 2 is a schematic configuration diagram of the dentition image generation system according to the embodiment.
[FIG. 3]

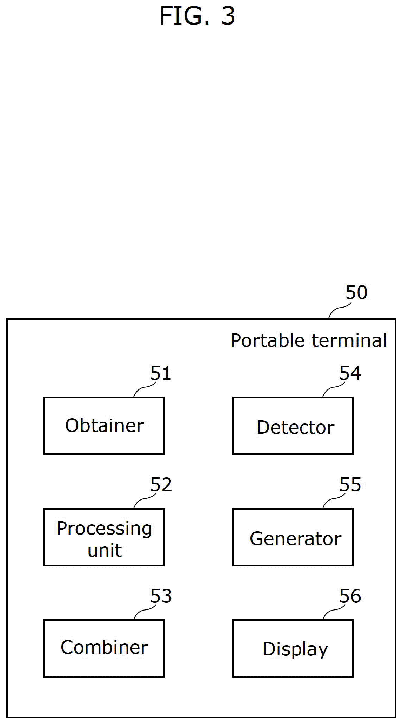

   FIG. 3 is a block diagram illustrating a functional configuration of a portable terminal according to the embodiment.
[FIG. 4]

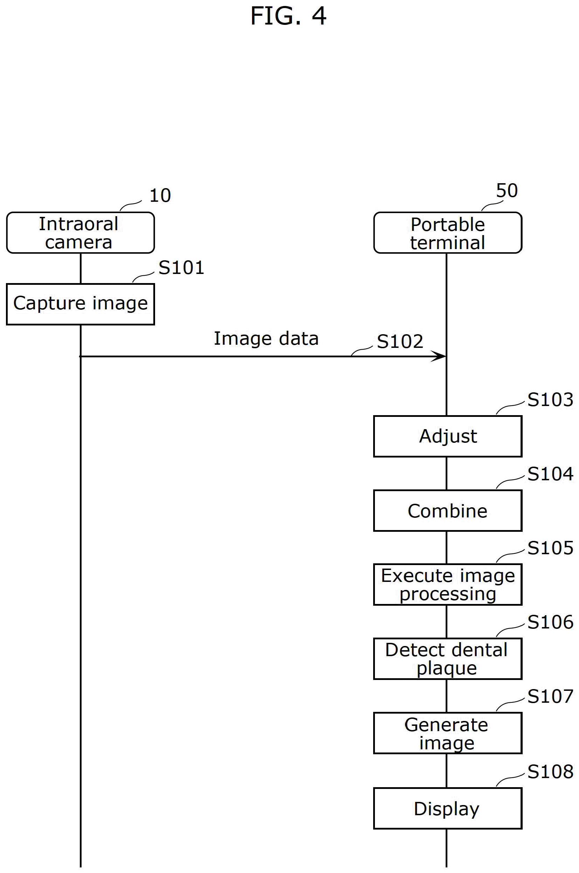

   FIG. 4 is a sequence diagram illustrating an operation of the dentition image generation system according to the embodiment.
[FIG. 5]

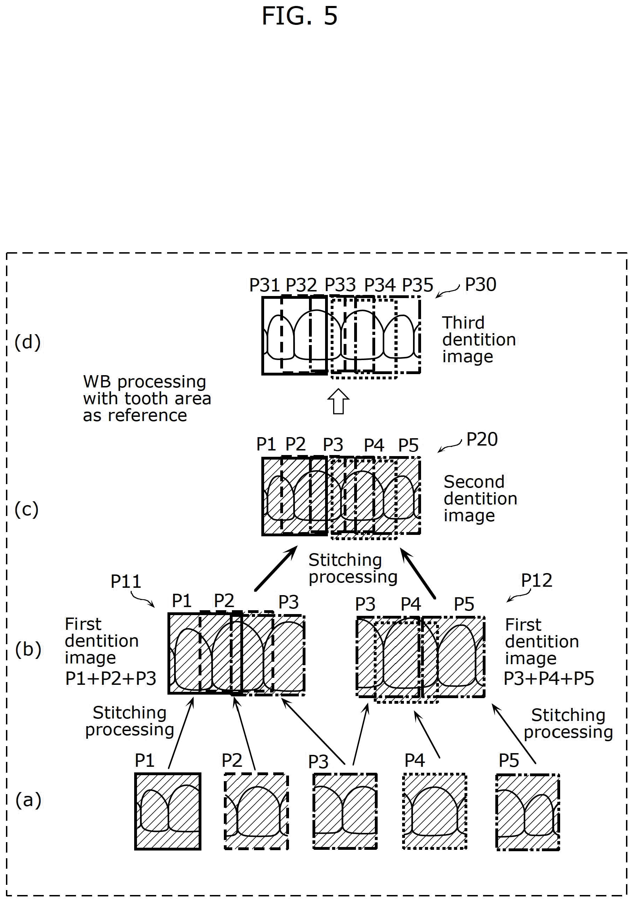

   FIG. 5 is a diagram for describing processing by the dentition image generation system according to the embodiment.

### [Description of Embodiments]

### (Circumstances Leading to the Present Disclosure)

Before describing the present disclosure, the circumstances leading to the present disclosure will be described.

Patent Literature 1 discloses that a light emitting diode (LED) having a center wavelength of 365 nm, 405 nm, or 470 nm is used as a light source of excitation light. When capturing an image of an affected area and the periphery of the affected area using such excitation light, the color tones of a tooth area without dental plaque and the periphery of the affected area in the obtained image becomes a color tone close to the light color of the excitation light. For this reason, the color tones of the tooth area without dental plaque and the periphery of the affected area deviate significantly from the actual color tones, which may cause a user to perceive an unnatural appearance in the obtained image. For example, when observing the condition of normal tissue around the affected area or when providing an explanation to a user, using the obtained image, the user may perceive an unnatural appearance because the color tones deviate significantly from the actual color tones.

Furthermore, when adjusting the excitation light emitter and the illumination light emitter in order to enhance visibility of dental plaque, a lesion area, and so on, reproducibility of color tones for each obtained image is low, which may cause the user to perceive an unnatural appearance in the obtained image.

In this manner, in the device of Patent Literature 1, the generating of a dentition image in which unnatural appearance is reduced can be improved upon. In view of this, the inventors of the present application have diligently studied a dentition image generation device, and so on, capable of generating a dentition image in which unnatural appearance is reduced, and have devised the dentition image generation device, and so on, described below.

A dentition image generation device according to a first aspect of the present disclosure includes: an obtainer that obtains a captured image obtained by capturing an image of a surface of dentition and dental plaque in an oral cavity irradiated with light including a blue-light wavelength range; a combiner that generates a dentition image obtained by combining a plurality of dentition block images that are partial dentition images based on the captured image; and a processing unit that performs white balance processing on the dentition image.

In this way, white balance adjustment using the appropriate gain can be performed on the dentition image obtained by combining a plurality of dentition block images. In other words, the color tone of the dentition image can be made to approach the actual color tone. Therefore, it is possible to generate the dentition image in which an unnatural appearance with respect to color tone is reduced.

Furthermore, for example, a dentition image generation device according to a second aspect is the dentition image generation device according to the first aspect, in which the processing unit may perform the white balance processing based on color information of a central tooth in the dentition image.

In this way, white balance adjustment using the appropriate gain can be performed on the central tooth. In other words, the color tone of particularly the central tooth in the dentition image can be made to approach the actual color tone. Therefore, for example, when the area on which the user focuses is an area including the central tooth, the plaque area in the area of focus can be effectively emphasized.

Furthermore, for example, a dentition image generation device according to a third aspect is the dentition image generation device according to the first aspect, in which the processing unit may perform the white balance processing based on color information of an entirety of a tooth area in the dentition image.

In this way, white balance adjustment using the appropriate gain (average gain) can be performed on the entirety of the teeth in the dentition image. In other words, the color tone of the teeth shown in the dentition image as a whole can be made to approach the actual color tone.

Furthermore, for example, a dentition image generation device according to a fourth aspect is the dentition image generation device according to any one of the first to third aspects, in which: the processing unit may adjust a blue level of a tooth area in each of the plurality of dentition block images, and the combiner may combine the plurality of dentition block images for which the blue level has been adjusted.

In this way, since the respective blue levels of the plurality of dentition block images can be made uniform, the color (color information) of the tooth area in the dentition image after WB processing can be made uniform. For example, it is possible to suppress an unnatural appearance that a user may perceive due to slight differences in the color of the respective tooth areas of the plurality of dentition block images (unnatural appearance due to non-uniform white colors).

Furthermore, for example, a dentition image generation device according to a fifth aspect is the dentition image generation device according to any one of the first to fourth aspects, that may further include: a detector that detects the dental plaque in an image that is based on the captured image; and a generator that generates the dentition image in which a dental plaque area detected by the detector is emphasized.

In this way, the dental plaque area in the dentition image can be emphasized. For example, when such a dentition image is to be presented to the user, the user can easily check the dental plaque area.

Furthermore, for example, a dentition image generation device according to a sixth aspect is the dentition image generation device according to the fifth aspect, that may further include: a display that displays the dentition image in which the dental plaque area is emphasized.

In this way, the dentition image in which the dental plaque area is emphasized can be presented to the user. For example, by capturing an image of dentition after brushing and identifying a dental plaque area, an area that was missed during brushing can be presented to the user.

Furthermore, for example, a dentition image generation device according to a seventh aspect is the dentition image generation device according to any one of the first to sixth aspects, in which the plurality of dentition block images may include an image obtained by capturing front teeth.

In this way, an image capturing teeth from a tooth on the right side (for example, a right molar) up to the front teeth and an image capturing teeth from a tooth on the left side (for example, a left molar) to the front teeth can be combined using the front teeth as a reference. Therefore, a dentition image from the tooth on the right side to the tooth on the left side can be generated.

Furthermore, for example, a dentition image generation device according to an eighth aspect is the dentition image generation device according to any one of the first to seventh aspects, in which the surface of the dentition may include a side surface of the dentition.

In this way, as a dentition image in which an unnatural appearance with respect to color tone is reduced, a dentition image obtained by capturing a side surface of the dentition can be generated.

Furthermore, for example, a dentition image generation device according to a ninth aspect is the dentition image generation device according to any one of the first to eighth aspects, in which the surface of the dentition may include an occlusal surface of the dentition.

In this way, as a dentition image in which an unnatural appearance with respect to color tone is reduced, a dentition image obtained by capturing an occlusal surface of the dentition can be generated.

Furthermore, for example, a dentition image generation device according to a tenth aspect is the dentition image generation device according to any one of the first to ninth aspects, that may further include a saturation enhancement processor that generates the dentition image in which saturation is enhanced, by: generating a converted image by converting the dentition image into an HSV image; identifying a specific pixel area in which one or more pixels satisfying at least one of a plurality of conditions are located, the one or more pixels being included in a plurality of pixels included in the converted image, the plurality of conditions being: that saturation is within a first predetermined range; that hue is within a second predetermined range; and brightness is within a third predetermined range; and performing saturation enhancement processing on the specific pixel area in the dentition image.

In this way, since a specific pixel area is identified as a dental plaque area in the dentition image, and saturation enhancement processing is performed on the specific pixel area, it is possible to generate a dentition image (a dentition image in which saturation S is emphasized) that further facilitates distinguishing of the dental plaque area.

Furthermore, for example, a dentition image generation device according to an eleventh aspect is the dentition image generation device according to any one of the first to tenth aspects, that may further include a gradation display processor that generates the dentition image including a gradation display by: generating a converted image by converting the dentition image into an HSV image; identifying a specific pixel area in which one or more pixels satisfying at least one of a plurality of conditions are located, the one or more pixels being included in a plurality of pixels included in the converted image, the plurality of conditions being: that saturation is within a first predetermined range; that hue is within a second predetermined range; and brightness is within a third predetermined range; detecting an accumulation level distribution of a fluorescent substance accumulated in the dental plaque, from a value of the brightness in the specific pixel area of the converted image; and performing gradation image processing on the specific pixel area in the dentition image, according to the accumulation level distribution of the fluorescent substance detected.

In this way, it is possible to generate a dentition image that can notify the concentration distribution of the fluorescent substance to the user, for example.

Furthermore, a dentition image generation method according to one aspect of the present disclosure includes: obtaining a captured image obtained by capturing an image of a surface of dentition and dental plaque in an oral cavity irradiated with light including a blue-light wavelength range; generating a dentition image obtained by combining a plurality of dentition block images that are partial dentition images based on the captured image; and performing white balance processing on the dentition image. Furthermore, a program according to one aspect of the present disclosure is a program for causing a computer to execute the above dentition image generation method.

In this way, it is possible to achieve the same results as with the above-described dentition image generation system.

It should be noted that these general or specific aspects may be implemented as a system, a method, an integrated circuit, a computer program, or a non-transitory computer-readable recording medium such as a CD-ROM or may be implemented as any combination of a system, a method, an integrated circuit, a computer program, and a recording medium. The program may be recorded in advance on a recording medium, or may be supplied to the recording medium via a wide-area communication network such as the Internet.

Moreover, the figures are schematic illustrations and are not necessarily precise depictions. Accordingly, for example, the scaling depicted in the figures is not necessarily uniform. Moreover, in the figures, elements that are substantially the same are assigned the same reference signs, and overlapping description is omitted or simplified.

Moreover, in the Specification, terms indicating relationships between elements such as identical, as well as numerical values and numerical value ranges, are not only expressions with strict meanings, but also expressions whose meanings include a substantially equivalent range having, for example, a difference of around several percent (or around 10%).

Moreover, in the Specification, unless otherwise noted, ordinal numerals such as "the first" and "the second" do not indicate the number or order of elements, but are used to avoid a mix-up between elements of the same kind and distinguish one from another.

### (Embodiment)

Hereinafter, a dentition image generation system and a dentition image generation method according to the present embodiment will be described with reference to FIG. 1 to FIG. 5.

### [1. Configuration of Dentition Image Generation System]

First, a configuration of a dentition image generation system including an intraoral camera according to the present embodiment will be described with reference to FIG. 1 to FIG. 3. FIG. 1 is a perspective view of intraoral camera 10 in the dentition image generation system according to the present embodiment.

As illustrated in FIG. 1, intraoral camera 10 includes a toothbrush-shaped case that can be handled with one hand. The case includes: head 10a which is put inside the oral cavity of a user when capturing an image of dentition; handle 10b which is designed to be held by the user; and neck 10c that connects head 10a and handle 10b.

Imaging unit 21 captures an image of a surface of dentition and dental plaque in an oral cavity irradiated with light including a blue-light wavelength range. The surface of the dentition includes at least one of a buccal (outer) side surface of the dentition, a lingual (inner) side surface of the dentition, and an occlusal surface of the dentition.

Imaging unit 21 is incorporated into head 10a and neck 10c. Imaging unit 21 includes an image sensor (not illustrated) and a lens (not illustrated) that are disposed on optical axis LA thereof.

The image sensor is, for example, an imaging device, such as a complementary metal-oxide-semiconductor (CMOS) sensor or a charge coupled device (CCD), and an image of a tooth is formed by the lens. The image sensor outputs a signal (image data) corresponding to the formed image to an external destination. A captured image captured by the image sensor is also referred to as an RGB image. Furthermore, the RGB image is an image sequence obtained by irradiating dentition with blue light, and may be, for example, an image sequence in which a side surface of the dentition is captured or an image sequence in which an occlusal surface of the dentition is captured. The image sequence includes, for example, one or more images (for example, time-series images) captured along an arrangement direction of the dentition. Furthermore, the side surface of the dentition may be a lingual side surface or a buccal side surface.

Imaging unit 21 may further include an optical filter that blocks light of a color emitted from an illuminator (illumination device) and transmits fluorescence emitted by dental plaque in response to exposure to the light. In the present embodiment, imaging unit 21 may include, as the optical filter, a blue-light blocking filter that blocks the light components of a blue wavelength contained in light incident on the image sensor. When teeth are irradiated with light including a blue-light wavelength range to detect dental plaque, if the light including the blue-light wavelength range is intensified to intensify the excitation fluorescence of the dental plaque, the blue pixel value becomes dominant compared to the red pixel value and the green pixel value, and thus the entire RGB image takes on a blue tinge. To deal with the issue, the blue-light blocking filter blocks part of the light including the blue-light wavelength range from the light yet to enter the image sensor. It should be noted that imaging unit 21 need not include a blue-light blocking filter.

Moreover, intraoral camera 10 includes first to fourth LEDs 23A to 23D as illuminators that irradiate a tooth that is the imaging target during capturing. First to fourth LEDs 23A to 23D emit light with a color (for example, light with a single color) that causes dental plaque that has been irradiated with the light to emit fluorescence. First to fourth LEDs 23A to 23D are, for example, blue LEDs that emit blue light including a wavelength having peak at 405 nm (an example of a predetermined wavelength). It should be noted that first to fourth LEDs 23A to 23D are not limited to blue LEDs as long as they are light sources that emit light including the blue-light wavelength range.

FIG. 2 is a schematic configuration diagram of the dentition image generation system according to the present embodiment. The dentition image generation system according to the present embodiment is generally configured such that imaging unit 21 captures fluorescence emitted by dental plaque in response to light from illuminator 23, and generates a panoramic image of a type in which teeth are aligned in a horizontal row by combining (concatenating and combining) a plurality of images (for example, first dentition block images described later) based on the captured RGB image. The panoramic image is an image representing a state of at least a part (for example, an entirety) of a mouth of the user.

As illustrated in FIG. 2, the dentition image generation system includes intraoral camera 10 and portable terminal 50.

Intraoral camera 10 includes hardware 20, signal processor 30, and communicator 40.

Hardware 20 is a physical element included in intraoral camera 10, and includes imaging unit 21, sensor 22, illuminator 23, and operation interface 24.

Imaging unit 21 generates image data by capturing an image of a tooth inside the oral cavity of the user. Imaging unit 21 can also be said to generate image data by capturing an image of a surface of dentition and dental plaque in the oral cavity irradiated with irradiation light of a predetermined wavelength that excites a fluorescent substance contained in the dental plaque. Imaging unit 21 receives a control signal from camera controller 31, performs an operation, such as image-capturing, in accordance with the received control signal, and outputs, to image processor 32, image data such as a video or a still image obtained through the image-capturing. Imaging unit 21 includes the above-described image sensor, optical filter, and lens. The image data is, for example, generated according to the light that has been transmitted through the optical filter. Moreover, the image data is an image showing teeth, but may be an image showing at least one tooth.

Sensor 22 detects external light entering the image-capturing area of an RGB image. For example, sensor 22 detects whether external light is entering the oral cavity. For example, sensor 22 is disposed near imaging unit 21. As with imaging unit 21, sensor 22 may be disposed in head 10a of intraoral camera 10, for example. That is, when imaging unit 21 captures an image, sensor 22 is located inside the oral cavity of the user.

Among a plurality of areas inside the oral cavity, illuminator 23 irradiates the area to be captured by imaging unit 21 with light. As is also known in a quantitative light-induced fluorescence method (QLF method), when irradiated with blue light, a substance called porphyrin excreted by bacteria in dental plaque fluoresces (excitation fluorescence) in a reddish pink color, and in the present embodiment, illuminator 23 irradiates the area to be captured by imaging unit 21 with blue light.

Illuminator 23 includes first to fourth LEDs 23A to 23D described above. For example, first to fourth LEDs 23A to 23D irradiate the image-capturing area with light rays in mutually different directions. In this way, the creation of shadows in the image-capturing area can be suppressed.

Each of first to fourth LEDs 23A to 23D is configured such that at least dimming is controllable. Each of first to fourth LEDs 23A to 23D may be configured such that dimming and color adjustment are controllable. First to fourth LEDs 23A to 23D are disposed to surround imaging unit 21.

The irradiation intensity (light emission intensity) of illuminator 23 is controlled according to the image-capturing area. The irradiation intensities of first to fourth LEDs 23A to 23D may be controlled uniformly, or may be controlled so as to be different from one another. It should be noted that the number of the LEDs of illuminator 23 is not limited to a particular number, and may be one or five or more. Moreover, illuminator 23 is not limited to including LEDs as light sources and may include another light source.

Operation interface 24 receives an operation from a user. Operation interface 24 is composed of, for example, a push button, or the like, but may be configured to receive an operation by sound.

Moreover, hardware 20 may further include a battery (e.g., a secondary battery) for supplying power to the elements of intraoral camera 10, a coil for use in wireless charging by an external charger connected to a commercial power supply, and an actuator necessary to perform at least one of composition adjustment or focus adjustment.

Signal processor 30 includes functional elements realized by using, for example, a central processing unit (CPU) or a microprocessor unit (MPU) that performs various processing tasks described later, and memory 35 such as read only memory (ROM) or random access memory (RAM) that stores programs for causing the functional elements to perform the various processing tasks. Specifically, signal processor 30 includes camera controller 31, image processor 32, controller 33, illumination controller 34, and memory 35.

Camera controller 31 is included in, for example, handle 10b of intraoral camera 10, and controls imaging unit 21. For instance, camera controller 31 controls at least one of the aperture or shutter speed of imaging unit 21 according to a control signal from image processor 32.

Image processor 32 is included in, for example, handle 10b of intraoral camera 10. Image processor 32 obtains an RGB image (image data) captured by imaging unit 21, performs image processing on the obtained RGB image, and outputs the RGB image which has undergone the image processing to camera controller 31 and controller 33. Moreover, image processor 32 may output the RGB image which has undergone the image processing to memory 35, to thereby store, in memory 35, the RGB image which has undergone the image processing.

Image processor 32 is, for example, configured as a circuit, and executes image processing, such as noise removal and outline enhancement processing, on an RGB image. Noise removal, outline enhancement processing, and so on, may be executed by portable terminal 50.

It should be noted that the RGB image (the RGB image that has undergone the image processing) outputted from image processor 32 may be transmitted to portable terminal 50 via communicator 40, and an image (for example, a third dentition image described later) based on the transmitted RGB image may be displayed on display 56 of portable terminal 50. In this way, the image based on the RGB image can be presented to the user.

Controller 33 is a control device that controls signal processor 30. Controller 33 controls the elements included in signal processor 30, based on the result of the detection of external light, and so on, by sensor 22, for example.

Illumination controller 34 is provided in, for example, handle 10b of intraoral camera 10, and controls turning on and off of first to fourth LEDs 23A to 23D. Illumination controller 34 is configured as a circuit, for example. When, for example, the user performs, on display 56 of portable terminal 50, an operation to activate intraoral camera 10, a corresponding signal is transmitted from portable terminal 50 to signal processor 30 via communicator 40. Illumination controller 34 of signal processor 30 turns on first to fourth LEDs 23A to 23D in accordance with the received signal.

Aside from the above programs, for example, the RGB image (image data) captured by imaging unit 21 is stored in memory 35. Memory 35 is realized by, for example, a semiconductor memory such as a ROM or a RAM, but is not limited thereto.

Communicator 40 is a wireless communication module for performing wireless communication with portable terminal 50. Communicator 40 is provided in, for example, handle 10b of intraoral camera 10, and performs wireless communication with portable terminal 50 in accordance with the control signal from signal processor 30. Communicator 40 performs, with portable terminal 50, wireless communication complying with an existing communication standard, such as Wi-Fi (registered trademark) or Bluetooth (registered trademark). An RGB image is transmitted from intraoral camera 10 to portable terminal 50 via communicator 40, and an operation signal is transmitted from portable terminal 50 to intraoral camera 10 via communicator 40.

For example, portable terminal 50 displays a dental plaque area in a second dentition image including two or more teeth, based on an RGB image obtained by irradiating teeth with light including the blue-light wavelength range and capturing a surface of dentition and dental plaque exhibiting a fluorescence reaction. Moreover, portable terminal 50 functions as the user interface of the dentition image generation system. Portable terminal 50 is an example of a dentition image generation device.

FIG. 3 is a block diagram illustrating a functional configuration of portable terminal 50 according to the present embodiment.

As illustrated in FIG. 3, portable terminal 50 includes obtainer 51, processing unit 52, combiner 53, detector 54, generator 55, and display 56. Portable terminal 50 includes a processor, memory, and so on. The memory is, for example, a ROM, a RAM, or the like, and can store programs to be executed by the processor. Obtainer 51, processing unit 52, combiner 53, detector 54, and generator 55 can be realized by, for example, a processor that executes programs stored in the memory. Portable terminal 50 may be realized as, for example, a smartphone or a tablet terminal capable of performing wireless communication.

Obtainer 51 obtains an RGB image from intraoral camera 10. Specifically, obtainer 51 obtains an image (image sequence) showing a plurality of teeth, generated by imaging unit 21. The RGB image is an image obtained by intraoral camera 10 irradiating teeth with light including the blue-light wavelength range and capturing an image of teeth exhibiting a fluorescence reaction. Obtainer 51 is configured to include, for example, a wireless communication module that performs wireless communication.

Here, the RGB image obtained by obtainer 51 takes on a blue tinge as a whole. This is because, when teeth are irradiated with light including the blue-light wavelength range to detect dental plaque, illuminator 23 intensifies the light including the blue-light wavelength range in order to intensify the excitation fluorescence of the dental plaque. In this way, a blue pixel value (B) becomes dominant compared to a red pixel value (R) and a green pixel value (G). In other words, color cast occurs in the RGB image obtained by obtainer 51. In this state, it is difficult to present the state of adhesion of dental plaque to a user in an easy-to-understand manner. In view of this, portable terminal 50 executes processing for presenting the state of adhesion of dental plaque to a user in an easy-to-understand manner, by performing predetermined image processing on the RGB image obtained by obtainer 51, as described below.

Processing unit 52 generates a plurality of first dentition block images, which are partial dentition images, from the RGB image (image sequence) generated by imaging unit 21, and performs image processing on an image based on the generated plurality of first dentition block images. In the present embodiment, processing unit 52 performs image processing on each of the plurality of first dentition block images and on each of a first dentition image or a second dentition image obtained by combining the plurality of first dentition block images.

A first dentition block image is an image obtained by cutting out, from an image captured at a certain image-capturing position, a predetermined range including the center of the angle of view. Each of the plurality of first dentition block images is an image based on a different one of images having different image-capturing positions (or image-capturing directions).

Furthermore, each of the plurality of first dentition block images is an image including at least one tooth or an interdental space. The first dentition block image may be, for example, an image obtained by capturing at least one tooth or an interdental space from the front. The size (image size) of the first dentition block images may be the same or may be different. Furthermore, in each of first dentition block images, at least a part of a tooth area shown in the first dentition block image overlaps with a part of a tooth area shown in at least one other first dentition block image.

It should be noted that an RGB image may itself be a first dentition block image, or first dentition block images may be generated by dividing the RGB image such that the first dentition block images partially overlap each other.

Processing unit 52 executes exposure correction processing on the plurality of first dentition block images, as image processing before the combining by combiner 53. In the exposure correction processing, the blue level of each tooth region is adjusted by multiplying an identical gain to each of the R, G, and B components. Processing unit 52 adjusts the blue level so that the blue levels of the respective tooth areas of the plurality of first dentition block images approach (for example, match) each other. For example, when the plurality of first dentition block images are arranged in dentition order, processing unit 52 adjusts the colors of respective teeth in the one or more other first dentition block images so as to be uniform with the color of a tooth (reference tooth) in the first dentition block image located at the center. The reference tooth may be, for example, a tooth located at the center of the angle of view in the RGB image captured by Imaging unit 21. since light from illuminator 23 may be unevenly irradiated at both ends of the angle of view, for example, adjusting the blue level makes it possible to reproduce a color as if illuminated in the same manner as the central tooth. It should be noted that the blue level adjustment may be performed only on the tooth area, or on the entirety of the first dentition block image (for example, an area including teeth and gums).

Furthermore, processing unit 52 executes white balance (WB) processing on the dentition image (the first dentition image or the second dentition image) as image processing after the combining by combiner 53. The WB processing is processing that adjusts the color balance in the image by multiplying different gains (white balance gains) to each of the R, G, and B components. For example, the WB processing is processing that adjusts the gains of at least two of the red, green, and blue components of the image to be processed so that the average red pixel value of a plurality of red pixel values, the average green pixel value of a plurality of green pixel values, and the average blue pixel value of a plurality of blue pixel values belonging to a plurality of pixels constituting the tooth area of the first dentition block image to be processed approach (for example, become equal to) each other.

Processing unit 52 executes the WB processing on the dentition image on the assumption that the tooth area shown in the dentition image is a white area. Processing unit 52 performs the WB processing on the dentition image, using the color (color information) of the tooth area shown in the dentition image as a reference. Processing unit 52 may execute the WB processing using the color (color information) of an arbitrary tooth (for example, a tooth shown at the center of the dentition image) as a reference, or may execute the WB processing using a statistical value (for example, an average value or a median value) of the color (color information) of the tooth area as a reference. For example, processing unit 52 may execute the WB processing by multiplying a gain calculated based on the arbitrary tooth or the statistical value, to the entire tooth area. Furthermore, when the blue levels have been adjusted before the combining by combiner 53, the blue levels in the dentition image obtained through the combining can be made uniform, making it possible to collectively perform white balance processing on the tooth area of the dentition image. Collectively means that the white balance processing uses a common gain.

As a result, the tooth area can be displayed as white (achromatic). In other words, it becomes easier to reproduce the actual color of the user's teeth. This leads to easier emphasizing of the dental plaque area in addition to reducing unnatural appearance in the image. Furthermore, when white balance processing is collectively performed, it is possible to standardize the color of the tooth area to the same color (white). For example, it is possible to suppress an unnatural appearance that a user may perceive regarding the color of the tooth area.

It should be noted that it is sufficient that processing unit 52 executes at least the WB processing among the adjustment of the blue level and the WB processing.

It should be noted that the tooth area shown in the dentition image may be a natural tooth area in the tooth shown in the dentition image. In other words, processing unit 52 may execute the WB processing on the assumption that the natural tooth area is a white area (for example, based on the color (color information) of the natural tooth area shown in the dentition image). The natural tooth area may be the natural tooth area in one tooth shown in the dentition image, or may be the natural tooth area in a predetermined tooth, or may be the natural tooth areas in a plurality of teeth. When the natural tooth areas in a plurality of teeth are used as a reference, a statistical value of the colors (for example, chromaticity) of the respective natural tooth areas in the plurality of teeth may be used for the WB processing. The statistical value is, for example, an average value, but may be a maximum value, a minimum value, a mode value, a median value, or the like.

It is known that when a natural tooth is irradiated with excitation light, excitation fluorescence is emitted from dentin and fluoresces green through enamel. Furthermore, it is known that a filling for a treated carious area (for example, a metal inlay) does not emit excitation fluorescence under blue LED light and is captured dark (at low luminance) by a camera. Furthermore, as described above, it is known that dental plaque (dental plaque area) fluoresces reddish pink (excitation fluorescence) when irradiated with blue light. From these facts, processing unit 52 can detect natural teeth excluding treated carious areas and dental plaque, from the dentition image.

It should be noted that processing unit 52 may further identify, from a first dentition block image, a first dentition image, or a dentition image (the second dentition image or the third dentition image), the type of tooth that is included in the image (for example, a specific tooth). Identifying the type of tooth may mean identifying whether the tooth is an incisor, a canine, or a molar, or may mean identifying whether the tooth is a central incisor, a lateral incisor, a canine, a first premolar, a second premolar, a first molar, a second molar, or a third molar (wisdom tooth). Furthermore, processing unit 52 may identify in which area of the oral cavity (upper jaw, lower jaw, left or right) the tooth is located. It should be noted that the method by which processing unit 52 identifies the type of tooth is not particularly limited, and may be, for example, a method using a machine learning model, a method using pattern matching, or any other known method. The machine learning model is a trained model that has been trained to output the type of tooth shown in an image when an image including a tooth is input. In the method using pattern matching, an image showing each tooth having a standard shape may be used as a reference, or an image of the teeth in the user's oral cavity captured in advance may be used as a reference. Furthermore, processing unit 52 may present information specifying a tooth that is the imaging target to the user before image-capturing, and determine that the tooth shown in an image obtained after the presentation is the tooth that is the imaging target, or may cause the user to input which tooth is included in the obtained image and determine that the inputted type of tooth is shown in the image.

Combiner 53 generates one dentition image (panoramic image) in which a plurality of teeth are arranged, by combining a plurality of first dentition block images in which the blue levels have been adjusted by processing unit 52. In the present embodiment, combiner 53 combines a plurality of dentition block images having adjusted blue levels using stitching processing, but the combining method is not limited thereto. The stitching processing may be performed, for example, using contours of teeth.

It should be noted that the stitching processing here is processing that generates one or more dentition images by combining a plurality of images having overlapping areas (here, the plurality of first dentition block images). In the stitching processing, first, the plurality of first dentition block images are arranged on a two-dimensional plane such that overlapping parts are aligned. Then, the plurality of first dentition block images are scaled (magnification-adjusted), positioned, and oriented so that at least a portion of feature points and surface points appear at the same location on a two-dimensional grid. In this manner, the plurality of first dentition block images are aligned with the positions where they are to be arranged. In other words, the plurality of first dentition block images are aligned to represent a series of adjacent teeth and are aligned so that positions of the teeth match.

It should be noted that, as the feature detection algorithm for detecting feature points, any type of feature detection algorithm may be used, including Scale-Invariant Feature Transform (SIFT) or Speeded-Up Robust Features (SURF). In the present embodiment, feature points may be provided on contour portions of the tooth area. Furthermore, the locations of surface points can be calculated by measuring a distance between focal points of viewpoints from which two first dentition block images including overlapping portions are respectively captured, extracting angles of optical axes at the respective viewpoints, and using triangulation based on common positions within the images.

It should be noted that, hereinafter, an example will be described in which combiner 53 generates, by combining, second dentition image P20 (see FIG. 5 described later) showing teeth from a left molar to a right molar, but the method of generating a second dentition image is not limited thereto, and it is sufficient to generate a second dentition image P20 including one or more teeth such as two or more teeth. In this case, combiner 53 may include, in second dentition image P20, information regarding the types of the one or more teeth or positions thereof in the oral cavity. This makes it possible to notify the user of which area in the oral cavity second dentition image P20 corresponds to. It should be noted that the types of teeth or the position in the oral cavity (for example, upper jaw, lower jaw, left or right area) are specified by processing unit 52.

Detector 54 executes detection of dental plaque (dental plaque area) on an image on which the WB processing has been executed (an example of an image based on the captured image). In the present embodiment, detector 54 executes detection of dental plaque, based on color information of the tooth area in the dentition image generated by combiner 53. The color information includes brightness V, hue H, and saturation S. For example, detector 54 detects dental plaque based on brightness V. For example, detector 54 detects an area having brightness V greater than or equal to a threshold as a dental plaque area.

It should be noted that detector 54 may detect dental plaque (dental plaque area) shown in the dentition image by using a machine learning model that has been trained to receive input of a dentition image on which the WB processing has been executed and output the dental plaque (dental plaque area) shown in the dentition image.

It should be noted that detector 54 may detect dental plaque before the combining by combiner 53, or after the combining by combiner 53 and before execution of the WB processing by processing unit 52. In other words, detector 54 may detect dental plaque using a color-cast image (an example of an image based on the captured image).

Generator 55 is a processing unit for generating a dentition image (for example, a third dentition image described later) in which the dental plaque area detected by detector 54 is emphasized. Generator 55 highlight displays the detected dental plaque area in the dentition image generated by combiner 53. For example, generator 55 superimposes highlight display on the dental plaque area of the dentition image.

It should be noted that, when detector 54 detects dental plaque before the WB processing, generator 55 may superimpose highlight display on the dental plaque area of the WB-processed third dentition image.

Display 56 is a display device included in portable terminal 50, and displays the image generated by generator 55. For example, display 56 may be realized by a liquid crystal display panel, or the like.

It should be noted that detector 54 and generator 55 may function as a saturation enhancement processor that performs saturation enhancement processing. For example, detector 54 may generate a converted image by converting a dentition image (for example, a third dentition image) generated through the combining by combiner 53 and subjected to the WB processing into an HSV image, and may identify a specific pixel area in which one or more pixels satisfying at least one of the following conditions are located, among the plurality of pixels included in the converted image: saturation S is within a first predetermined range (for example, at least 30 and at most 80 in 8-bit representation), hue H is within a second predetermined range (for example, at least 140 and at most 170 in 8-bit representation), and brightness V is within a third predetermined range (for example, at least 100 and at most 180 in 8-bit representation). Furthermore, generator 55 may generate a dentition image in which saturation S is enhanced by performing saturation enhancement processing on the specific pixel area in the dentition image (for example, the third dentition image). The HSV image is generated, for example, by converting a color space of the dentition image into an HSV space. Furthermore, display 56 may display the dentition image on which the saturation enhancement processing has been performed.

The first predetermined range, the second predetermined range, and the third predetermined range may be specified by comparing the actual dental plaque area and tooth area with the HSV image, and are not limited to the above numerical ranges. For example, the ranges of values of brightness V, hue H, and saturation S can be determined by administering a plaque disclosing agent and comparing with the degree of staining by the plaque disclosing agent.

It should be noted that detector 54 and generator 55 may function as a gradation display processor that performs gradation image processing according to a concentration distribution (accumulation level distribution) of a fluorescent substance. For example, detector 54 may detect a concentration distribution of the fluorescent substance accumulated in dental plaque, from a value of brightness V in the specific pixel area of the converted image. Furthermore, generator 55 may generate a dentition image (for example, a third dentition image) including gradation display, by performing gradation image processing on the specific pixel area in the dentition image according to the detected concentration distribution of the fluorescent substance.

In a process in which blue light passes through each layer, porphyrin in dental plaque is excited and red fluorescence is generated. Furthermore, the fluorescence intensity is considered not to reflect a current bacterial flora but to indicate accumulation of the fluorescent substance (porphyrin). In other words, as the accumulation of the fluorescent substance increases, the red fluorescence becomes stronger. That is, as dental plaque matures, the accumulation level of porphyrin increases. Therefore, fluorescence intensity of mature dental plaque is stronger than fluorescence intensity of young dental plaque.

Detector 54 detects the accumulation level (concentration or density) of the fluorescent substance by comparing intensity of red fluorescence per unit area of the dental plaque area.

As described above, a dental plaque area may be extracted from one or more pixels satisfying at least one of the following conditions in an HSV image: saturation S is within the first predetermined range; hue H is within the second predetermined range; and brightness V is within the third predetermined range.

Furthermore, in an image on which the WB processing has been executed, in a dental plaque area, brightness V is determined by the value of R regardless of hue H and saturation S.

Furthermore, it is known that the fluorescence wavelength of porphyrin, which is a fluorescent substance in dental plaque, is 600 nm to 740 nm, and that the peak fluorescence wavelength is 630 nm. That is, porphyrin concentration accumulated in a dental plaque area can be evaluated by detecting the value of brightness V in an HSV image of the dental plaque area.

### [2. Operation of Dentition Image Generation System]

Next, the operation of the dentition image generation system configured as described above will be described with reference to FIG. 4 and FIG. 5. FIG. 4 is a sequence diagram illustrating the operation (dentition image generation method) of the dentition image generation system according to the present embodiment. The processing illustrated in FIG. 4 is executed by portable terminal 50. It should be noted that the processing illustrated in FIG. 4 is, for example, processing performed in real time, and is performed per frame or every time image data of a plurality of frames is obtained. FIG. 5 is a diagram for describing processing by the dentition image generation system according to the present embodiment. It should be noted that, in FIG. 5, an image having a color cast is indicated by diagonal hatching.

As illustrated in FIG. 4, image data is generated by a user capturing teeth and gums in the user's oral cavity using intraoral camera 10 (S101). This image data is, for example, image data obtained by irradiating the teeth with light including the blue-light wavelength range and capturing teeth exhibiting a fluorescence reaction.

Next, communicator 40 of intraoral camera 10 transmits the captured image data to portable terminal 50, and obtainer 51 of portable terminal 50 obtains the image data (S102). The image data may be video or may be one or more still images. Moreover, when the image data is a video or a plurality of still images, image data may be transmitted for each frame of the video or for each still image. It should be noted that, when the image data is a video, image data may be transmitted on a multi-frame basis.

Moreover, transmission of image data may be performed in real time or the image data may be transmitted in a single transmission after a series of image-capturing is performed (e.g., after capturing images of all the teeth inside the oral cavity).

Processing unit 52 of portable terminal 50 adjusts the blue level of the received image data (S103). Processing unit 52 may multiply different gains to respective R, G, and B components so that blue levels of tooth areas in each of a plurality of first dentition block images, which are partial dentition images based on the image data, approach each other.

Each of a plurality of first dentition block images P1 to P5 illustrated in (a) in FIG. 5 is an image on which WB processing has not been executed. Furthermore, each of the plurality of first dentition block images P1 to P5 is an image in which the blue level has been adjusted.

Referring again to FIG. 4, next, combiner 53 of portable terminal 50 combines the plurality of first dentition block images P1 to P5 on which WB processing has not been executed and in which the blue level has been adjusted (S104).

As illustrated in (a) and (b) in FIG. 5, combiner 53 executes stitching processing on first dentition block images P1, P2, and P3, to thereby generate first dentition image P11 including first dentition block images P1 and P2 and at least a part of first dentition block image P3. Furthermore, combiner 53 executes stitching processing on first dentition block images P3, P4, and P5, to thereby generate first dentition image P12 including at least a part of first dentition block image P3, and first dentition block images P4 and P5. First dentition images P11 and P12 are images in which at least a part of tooth areas overlaps. In the example in FIG. 5, at least a part of the tooth area of first dentition block image P3 overlaps in first dentition images P11 and P12.

Furthermore, processing unit 52 executes stitching processing such that a central portion of an angle of view of each of the first dentition block images to be combined is included in the first dentition image. Describing this using first dentition image P11 as an example, processing unit 52 executes stitching processing such that the central portion of the angle of view of each of first dentition block images P1 to P3 is included in first dentition image P11. First dentition image P11 generated in the above manner is an image in which light is applied to each tooth from the front, and thus shadows are less likely to occur and interdental spaces can be more easily seen.

Here, first dentition block image P3 is an image including two front teeth of the lower teeth and may be, for example, an image including the interdental space between the two front teeth. In this case, first dentition image P11 may be, for example, a panoramic image from a left molar to the front teeth, and first dentition image P12 may be, for example, a panoramic image from a right molar to the front teeth.

It should be noted that an image including the interdental space between the front teeth may be, for example, an image captured after an announcement such as "Please capture front teeth" is made at the time of capturing by imaging unit 21, or may be an image for which input indicating that it is an image of the front teeth is provided by the user or the like.

It should be noted that the number of first dentition images generated by combiner 53 is not particularly limited and may be three or more.

Then, combiner 53 executes stitching processing on the two first dentition images P11 and P12 to thereby generate second dentition image P20. Second dentition image P20 is a panoramic image obtained by combining the plurality of first dentition block images P1 to P5. Second dentition image P20 is a panoramic image showing at least a part of dentition in the oral cavity of the user and may be, for example, a panoramic image showing teeth from a left molar to a right molar of the user. Second dentition image P20 is an example of a dentition image after combining.

It should be noted that combiner 53 may directly generate second dentition image P20 based on the plurality of first dentition block images P1 to P5. In other words, first dentition images P11 and P12 need not be generated.

Referring again to FIG. 4, next, processing unit 52 of portable terminal 50 executes image processing on second dentition image P20 resulting from the combining by combiner 53 (S105). In step S105, processing unit 52 executes at least WB processing on second dentition image P20.

As illustrated in (c) and (d) in FIG. 5, processing unit 52 generates third dentition image P30 by executing WB processing on second dentition image P20, with the color information of the tooth area of second dentition image P20 as a reference. Processing unit 52 may execute the WB processing with the color information of the central tooth in second dentition image P20 (for example, the teeth shown in first dentition block image P3) as a reference. Processing unit 52 may, for example, execute the WB processing with the color information of only the central tooth in second dentition image P20 (for example, the teeth shown in first dentition block image P3) as a reference.

It should be noted that third dentition image P30 is composed of the plurality of second dentition block images P31 to P35. Second dentition block image P31 corresponds to an image in which white balance processing has been executed on first dentition block image P1; second dentition block image P32 corresponds to an image in which white balance processing has been performed on first dentition block image P2; second dentition block image P33 corresponds to an image in which white balance processing has been performed on first dentition block image P3; second dentition block image P34 corresponds to an image in which white balance processing has been performed on first dentition block image P4; and second dentition block image P35 corresponds to an image in which white balance processing has been performed on first dentition block image P5.

Referring again to FIG. 4, detector 54 of portable terminal 50 detects dental plaque in third dentition image P30 (S106). Detector 54 detects the presence or absence of dental plaque, but may, for example, detect the concentration distribution of a fluorescent substance, that is, the accumulation level of dental plaque.

Next, generator 55 of portable terminal 50 generates an image in which a dental plaque area where the dental plaque detected by detector 54 is attached is emphasized (S107). In the present embodiment, generator 55 generates an image in which a highlight display indicating the dental plaque area is superimposed on third dentition image P30. However, generator 55 may generate an image in which a gradation display that is in accordance with the concentration distribution of the fluorescent substance is superimposed on third dentition image P30.

Next, display 56 of portable terminal 50 displays the image generated by generator 55 (S108).

By using such a dentition image generation system, the user can capture an image of the user's own oral cavity using intraoral camera 10 and check the state of the inside of the oral cavity displayed on portable terminal 50 as a panoramic image. In addition, because the concentration distribution of the fluorescent substance is shown in the displayed panoramic image, the user can easily confirm the health condition of the user's teeth.

Furthermore, since portable terminal 50 executes white balance processing, portable terminal 50 can generate third dentition image P30 in which the dental plaque area is superimposed (for example, emphasized), regardless of the color of the light emitted by illuminator 23 of intraoral camera 10.

Furthermore, portable terminal 50 may, for example, generate a three-dimensional model of a plurality of teeth in the oral cavity, from a plurality of captured items of image data. Furthermore, portable terminal 50 may display an image based on the generated three-dimensional model.

It should be noted that detector 54 may detect dental plaque in first dentition images P11 and P12 instead of detecting dental plaque in second dentition image P20.

Moreover, although an example in which portable terminal 50 performs processing of images of teeth has been described here, intraoral camera 10 may perform part or all of this processing.

### (Other Embodiments)

Although the dentition image generation system according to an embodiment of the present disclosure has been described above, the present disclosure is not limited to this embodiment.

For example, although an example in which intraoral camera 10 mainly intended to capture images of teeth is used has been described in the above embodiment, intraoral camera 10 may be an intraoral care device including a camera. For example, intraoral camera 10 may be an intraoral cleaning device, or the like, that includes a camera.

Furthermore, although portable terminal 50 has been exemplified as an information terminal of the user in the above embodiment, the information terminal may be a stationary information terminal.

Furthermore, in the above embodiment, the plurality of first dentition block images may be, for example, images captured after an announcement of the position of the teeth or the type of the teeth is made at the time of image-capturing by imaging unit 21, or may be images for which the position of the tooth shown in the image has been input by the user or the like.

Furthermore, each of the processing units included in the dentition image generation system according to the above embodiments is implemented typically as an LSI circuit which is an integrated circuit. These may take the form of individual chips, or may be partially or entirely packaged into a single chip.

Furthermore, circuit integration is not limited to an LSI, and thus may be implemented as a dedicated circuit or a general-purpose processor. A field programmable gate array (FPGA) that allows for programming after the manufacture of an LSI, or a reconfigurable processor that allows for reconfiguration of the connection and the setting of circuit cells inside an LSI may be employed.

Moreover, in the above embodiments, each of the elements may be configured as dedicated hardware or may be realized by executing a software program suitable for the element. Alternatively, the elements may be implemented by a program executor such as a CPU or a processor reading out and executing the software program recorded on a recording medium such as a hard disk or a semiconductor memory.

Moreover, the allocation of the functional blocks illustrated in each block diagram is a mere example. A plurality of functional blocks may be incorporated into one functional block. One functional block may be divided into a plurality of functional blocks. A part of the function may be transferred from one functional block to another functional block. Moreover, the functions of a plurality of functional blocks having similar functions may be processed by single hardware or software in a parallelized or time-divided manner.

Moreover, portable terminal 50 according to the above embodiments may be implemented as a single device or a plurality of devices. When portable terminal 50 is implemented as a plurality of devices, the elements of portable terminal 50 may be divided among the devices in any way. For example, among the functions of portable terminal 50, at least one or more functions may be realized by intraoral camera 10 (for example, signal processor 30). When portable terminal 50 is implemented as a plurality of devices, the communication method between the devices is not limited to a particular method, and the communication may be wireless or wired communication. Moreover, a combination of wireless communication and wired communication may be used between the devices.

Furthermore, the present disclosure may be implemented as a dentition image generation method to be executed by the dentition image generation system. Furthermore, the present disclosure may be implemented as an intraoral camera, a portable terminal, or a cloud server included in the dentition image generation system.

Moreover, the order in which the steps in the sequence diagram are performed is provided as an example to specifically describe the present disclosure, and the steps may be performed in an order different from the above order. Furthermore, one or more of the above steps may be implemented simultaneously (in parallel) with another step.

Moreover, one aspect of the present disclosure may be a computer program for causing a computer to execute the characteristic steps included in the dentition image generation method illustrated in FIG. 4.

Moreover, for example, the program may be a program to be executed by the computer. Moreover, an aspect of the present disclosure may be a non-transitory computer-readable recording medium having such a program recorded thereon. For example, such a program may be distributed or circulated by being recorded on the recording medium. For example, by installing the distributed program onto a device including another processor, and causing the processor to execute the program, it is possible to cause the device to perform each processing task described above.

Although the dentition image generation system, and so on, according to one or more aspects has been described based on the above embodiments, the present disclosure is not limited to the above embodiments. The scope of the one or more aspects may thus include forms achieved by making various modifications to the above embodiments that can be conceived by those skilled in the art, as well forms achieved by combining elements in different embodiments, without materially departing from the spirit of the present disclosure.

### [Industrial Applicability]

The present disclosure can be applied to a dentition image generation system.

### [Reference Signs List]

10 intraoral camera
10a head
10b handle
10c neck
20 hardware
21 imaging unit
22 sensor
23 illuminator
23A first LED
23B second LED
23C third LED
23D fourth LED
24 operation interface
30 signal processor
31 camera controller
32 image processor
33 controller
34 illumination controller
35 memory
40 communicator
50 portable terminal (dentition image generation device)
51 obtainer
52 processing unit
53 combiner
54 detector (saturation enhancement processor, gradation display processor)
55 generator (saturation enhancement processor, gradation display processor)
56 display
P1, P2, P3, P4, P5 first dentition block image
P11, P12 first dentition image
P20 second dentition image (dentition image)
P30 third dentition image
P31, P32, P33, P34, P35 second dentition block image

## Claims

1. A dentition image generation device comprising:
an obtainer that obtains a captured image obtained by capturing an image of a surface of dentition and dental plaque in an oral cavity irradiated with light including a blue-light wavelength range;
a combiner that generates a dentition image obtained by combining a plurality of dentition block images that are partial dentition images based on the captured image; and
a processing unit that performs white balance processing on the dentition image.

2. The dentition image generation device according to claim 1, wherein
the processing unit performs the white balance processing based on color information of a central tooth in the dentition image.

3. The dentition image generation device according to claim 1, wherein
the processing unit performs the white balance processing based on color information of an entirety of a tooth area in the dentition image.

4. The dentition image generation device according to any one of claims 1 to 3, wherein
the processing unit adjusts a blue level of a tooth area in each of the plurality of dentition block images, and
the combiner combines the plurality of dentition block images for which the blue level has been adjusted.

5. The dentition image generation device according to any one of claims 1 to 3, further comprising:
a detector that detects the dental plaque in an image that is based on the captured image; and
a generator that generates the dentition image in which a dental plaque area detected by the detector is emphasized.

6. The dentition image generation device according to claim 5, further comprising:
a display that displays the dentition image in which the dental plaque area is emphasized.

7. The dentition image generation device according to any one of claims 1 to 3, wherein
the plurality of dentition block images include an image obtained by capturing front teeth.

8. The dentition image generation device according to any one of claims 1 to 3, wherein
the surface of the dentition includes a side surface of the dentition.

9. The dentition image generation device according to any one of claims 1 to 3, wherein
the surface of the dentition includes an occlusal surface of the dentition.

10. The dentition image generation device according to any one of claims 1 to 3, further comprising:
a saturation enhancement processor that generates the dentition image in which saturation is enhanced, by:
generating a converted image by converting the dentition image into an HSV image;
identifying a specific pixel area in which one or more pixels satisfying at least one of a plurality of conditions are located, the one or more pixels being included in a plurality of pixels included in the converted image, the plurality of conditions being: that saturation is within a first predetermined range; that hue is within a second predetermined range; and brightness is within a third predetermined range; and
performing saturation enhancement processing on the specific pixel area in the dentition image.

11. The dentition image generation device according to any one of claims 1 to 3, further comprising:
a gradation display processor that generates the dentition image including a gradation display by:
generating a converted image by converting the dentition image into an HSV image;
identifying a specific pixel area in which one or more pixels satisfying at least one of a plurality of conditions are located, the one or more pixels being included in a plurality of pixels included in the converted image, the plurality of conditions being: that saturation is within a first predetermined range; that hue is within a second predetermined range; and brightness is within a third predetermined range;
detecting an accumulation level distribution of a fluorescent substance accumulated in the dental plaque, from a value of the brightness in the specific pixel area of the converted image; and
performing gradation image processing on the specific pixel area in the dentition image, according to the accumulation level distribution of the fluorescent substance detected.

12. A dentition image generation method comprising:
obtaining a captured image obtained by capturing an image of a surface of dentition and dental plaque in an oral cavity irradiated with light including a blue-light wavelength range;
generating a dentition image obtained by combining a plurality of dentition block images that are partial dentition images based on the captured image; and
performing white balance processing on the dentition image.

13. A program for causing a computer to execute the dentition image generation method according to claim 12.
